# EUROPEAN PATENT APPLICATION

(11) **EP 1 422 677 A2**
(43) Date of publication of application: **26.05.2004**
(21) Application number: 03257089.7
(22) Date of filing: 11.11.2003
(51) Int. Cl.: G08C 17/02

(54) **Living body information measuring system**

(30) Priority: 19.11.2002 JP 2002335284; 08.01.2003 JP 2003002151; 26.09.2003 JP 2003334643
(71) Applicant: Seiko Instruments Inc., Chiba-shi, Chiba (JP)
(72) Inventor: Iijima, Ryuji, c/o Seiko Instruments Inc., Chiba-shi Chiba (JP); Moriya, Koichi, c/o Seiko Instruments Inc., Chiba-shi Chiba (JP)
(74) Representative: Sturt, Clifford Mark

(57) **Abstract**

A Living Body Information Measuring System comprising a data collecting device and a measuring device, for saving the power consumed in the measuring device and preventing a data transmission delay of the measuring device in an emergency, is described.

The data collecting device calculates the "transfer schedule time of the next data transfer request command" in the data transfer request command, and varies the value of the "transfer schedule time of the next data transfer request command" according to the tendency of the transferred data and the time zone.

The measuring device controls to cut off the power of the receiver, until this "transfer schedule time of the next data transfer request command", after transferring the requested data.

## Description

The present invention relates to power saving of a measuring system having a wireless communication function.

There is a system including a measuring device that has a wireless communication function and transmits data when receiving a wireless request from the outside, for saving power. Further, there is an intermittent receiving method for saving power of a mobile communication system.

In a system where a measuring device transmits data when receiving a wireless transmission request from the outside, for saving power, though the power can be saved by holding a circuit of sampling data within the measuring device turn off until receiving the wireless transmission request, it is always necessary to turn on the power of a receiver for receiving the wireless transmission request, which consumes the power, thereby failing to save the power of a portion around the receiver. Further, while the measuring device is moving and measuring, it is always necessary to turn on the power of a receiver so that the measuring device can receive a data transfer request command even when after having got out of range of the radio waves from the data collecting device and the like, during which it couldn't receive the data transfer request command from the data collecting device, and it enters within range of the radio wave again, and therefore, the power is consumed for the above.

Further, since it is impossible to change the sampling intervals of the sensor data in the above case, the power consumption by a sensor circuit and the consumption of a temporary storing memory for the sensor data are great.

Further, when it returns in a position to receive the data transfer request command thereafter, the sampling data during the interval when it could not receive the data transfer request command, cannot be transferred to the data collecting device.

In the intermittent receiving method used in a mobile communication system, since the intermittent receiving interval is determined regardless of the tendency of the measurement data of the measuring device, when data is deviated from the correct value, the data reaches the data collecting device so late and it can't perform the minute monitoring at a necessary time disadvantageously. Further, there occurs a transfer delay of the urgent data at the data abnormal time.

In consideration of the above situation, the present invention provides a measuring system capable of closely monitoring the data on the side of the data collecting device in case of necessity and preventing from a transfer delay of the data in case of an emergency with a little consumption power in a wireless device, by including the "transfer schedule time of the next data transfer request command" in the data transfer request command from the data collecting device, varying the calculated value of the "transfer schedule time of the next data transfer request command" according to the tendency of the data transferred to the data collecting device and the time zone, and cutting off the power of the receiver in the measuring device, until the "transfer schedule time of the next data transfer request command", after wireless transmission of the requested data.

In order to realize the above, in the measuring system of the invention, the data collecting device has the command transfer schedule time calculating means for calculating the "transfer schedule time of the next data transfer request command" and the command creating means for including the value of the "transfer schedule time of the next data transfer request command" in the this data transfer request command, while the measuring device has the controlling means for cutting off the power of the receiver of the measuring device until the "transfer schedule time of the next data transfer request command", after transmitting the data requested by this data transfer request command.

The command transfer schedule time calculating means calculates the "transfer schedule time of the next data transfer request command", according to the tendency of the data transferred to the data collecting device.

Concretely, the command transfer schedule time calculating means sets the "transfer schedule time of the next data transfer request command" at a time closer to this data transfer request command transmission time, according as the data transferred to the data collecting device approaches the predetermined upper limit or lower limit, and sets it at a time far away therefrom according as it approaches the medium value of the upper limit and the lower limit.

The command transfer schedule time calculating means sets the "transfer schedule time of the next data transfer request command" at a time closer to this data transfer request command transmission time according as the dispersion of the data transferred to the data collecting device is larger, and sets it at a time far away therefrom according as the dispersion of the data is smaller.

The command transfer schedule time calculating means sets the "transfer schedule time of the next data transfer request command" at a time closer to this data transfer request command transmission time according as the variation rate of the data transferred to the data collecting device is larger, and sets it at a time far away therefrom according as the variation rate of the data is smaller.

The command transfer schedule time calculating means varies the "transfer schedule time of the next data transfer request command" according to the time zone in a day.

The measuring device has the judging means for transmitting the urgent information data to the data collecting device, at arbitrary timing, not according to the data transfer request command transmitted from the data collecting device, when the data detected by the sensor is abnormal value.

The measuring device has the judging means for transmitting the urgent information data to the data collecting device, at arbitrary timing, not according to the data transfer request command transmitted from the data collecting device, when the data detected by the sensor exceeds the predetermined upper limit or lower limit.

When the measuring device cannot receive the data transfer request command from the data collecting device for a predetermined period of time, the measuring device can save the power consumption and the memory consumption when it is out of range of the radio wave from the data collecting device, sample the sensor data in the above case, and transfer the data after return into range of the radio wave, by changing the operation of the receiver of the measuring device to the intermittent receiving operation and lengthening the sampling intervals of the sensor.

Therefore, the measuring system of the invention has the operation change judging means for changing the operation of the receiver of the measuring device to the intermittent receiving operation and lengthening the sampling intervals of the sensor when it cannot receive the data transfer request command from the data collecting device for a predetermined period of time, and the storing means for storing the sampling data during the period where it cannot receive the data transfer request command from the data collecting device, into a memory.

Further, it has the operation return judging means for changing the operation of the receiver to the continuous receiving operation and returning the sampling intervals of the sensor to the ordinal state, when the measuring device can receive the data transfer request command from the data collecting device, during the intermittent receiving operation because of the above factor, and the transmitting means for transmitting the current sampling data and the data sampled during the intermittent receiving operation and stored in the memory, to the data collecting device.

Further, it has the changing means for changing the intermittent receiving time intervals and the sampling intervals of the sensor according to the tendency of the data detected by the sensor, during the intermittent receiving operation because of the above factor.

Concretely, the changing means sets the intermittent receiving time intervals and the sampling intervals of the sensor at shorter periods than the predetermined reference values at the intermittent receiving time, according as the data detected by the sensor approaches the predetermined upper limit or lower limit, and sets the above intervals close to the reference values at the intermittent receiving time according as the above data approaches the medium value of the upper limit and the lower limit.

The changing means sets the intermittent receiving time intervals and the sampling intervals of the sensor at shorter periods than the predetermined reference values at the intermittent receiving time, according as the dispersion of the data detected by the sensor is larger, and sets the above intervals close to the reference values at the intermittent receiving time according as the dispersion of the data is smaller.

The changing means sets the intermittent receiving time intervals and the sampling intervals of the sensor at shorter periods than the predetermined reference values at the intermittent receiving time, according as the variation rate of the data detected by the sensor is larger and sets the above intervals close to the reference values at the intermittent receiving time, according as the variation rate of the data is smaller.

The changing means varies the intermittent receiving time intervals and the sampling intervals of the sensor depending on the time zone in a day.

Instead of creating the "transfer schedule time of the next data transfer request command" in the data collecting device, there is a mode of creating the corresponding schedule time value on the side of the measuring device and therefrom transmitting it to the data collecting device. This schedule time value is, hereinafter, referred to as "next data transmission schedule time".

The data collecting device determines the "transfer schedule time of the next data transfer request command" based on the "next data transmission schedule time" transmitted from the measuring device.

Namely, the data collecting device can transmit the data transfer request command to the measuring device at the "next data transmission schedule time" value and later.

The measuring device can cut off the power of the wireless transmitter/receiver, until the "next data transmission schedule time", after the data transmission, hence to realize the power saving.

The measuring device calculates the "next data transmission schedule time" according to the tendency of the data detected by the sensor.

According as the data detected by the sensor approaches the predetermined upper limit or lower limit, the "next data transmission schedule time" is set at a time close to this data transmission time, and according as the data approaches the medium value of the upper limit and the lower limit, it is set at a time far away therefrom.

According as the dispersion of the data detected by the sensor is larger, the "next data transmission schedule time" is set at a time close to this data transmission time, while according as the dispersion of the data is smaller, the above time is set at a time far away therefrom.

According as the variation rate of the data detected by the sensor is larger, the "next data transmission schedule time" is set at a time close to this data transmission time, while according as the variation rate of the data is smaller, the above time is set at a time far away therefrom.

Further, the "next data transmission schedule time" is varied depending on the time zone in a day.

Embodiments of the present invention will now be described by way of further example only and with reference to the accompanying drawings, in which:-
Fig. 1 is a block diagram showing the construction of the hardware of the measuring device according to one embodiment of the invention;
Fig. 2 is a block diagram showing the construction of the hardware of the data collecting device according to one embodiment of the invention;
Fig. 3A is a view showing the outline of the wireless transmitter/receiver according to the invention;
Fig. 3B is a view showing the data transfer request command packet according to the invention;
Fig. 3C is a view showing the data packet according to the invention;
Fig. 4 is a timing chart of wireless transmission/reception and receiver's power control according to the invention;
Fig. 5 is a flow chart of the processing of the data collecting device according to the invention;
Fig. 6 is a flow chart of the data reading processing of the data collecting device according to the invention;
Fig. 7 is a flow chart of the data sampling processing of the measuring device according to the invention;
Fig. 8 is a flow chart of the data transmitting/receiving processing of the measuring device according to the invention;
Fig. 9A is a timing chart of transition between continuous reception and intermittent reception of a receiver of the measuring device according to the invention;
Fig. 9B is a timing chart of the measuring device receiving operation according to the invention.
Fig. 9C is a timing chart of the measuring device receiver's power on/off operation according to the invention.
Fig. 9D is a timing chart of the measuring device sampling operation to the invention.
Fig. 10 is a flow chart showing the judgment processing of transition to the intermittent receiving operation of the measuring device according to the invention;
Fig. 11 is a flow chart showing the changing processing of intermittent receiving interval of the measuring device and sensor sampling interval according to the invention;
Fig. 12 is a view showing the data packet in case of providing the measuring device with the function of calculating the next data transmission schedule time according to the invention ;and
Fig. 13 is a flow chart showing the processing of the measuring device in the case of providing the measuring device with the function of calculating the next data transmission schedule time according to the invention.

Hereinafter, the present invention will be described in detail with reference to the drawings. However, this invention is not restricted to the form of the embodiment.

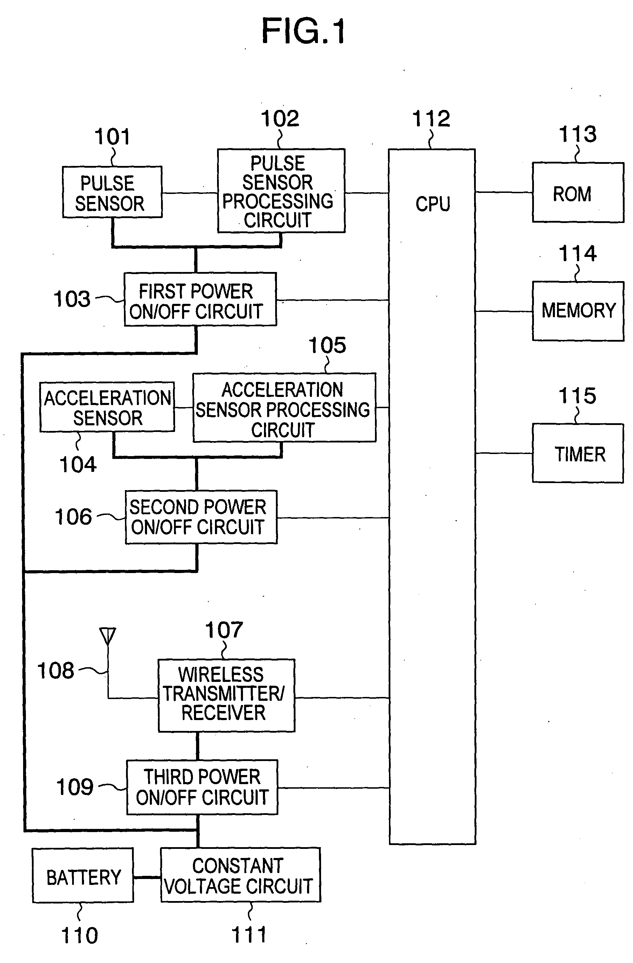

Fig. 1 is a view showing a hardware structure of a measurement device according to one embodiment of the invention. Here, a thin line indicates a signal line and a heavy line indicates a power line.

The measurement device of this embodiment is to be attached to an animal or a human body so as to detect living body information, which includes various sensors for detecting the living body information. Here, the case of providing it with a pulse sensor 101 for detecting pulse and an acceleration sensor 104 for detecting acceleration of a person to be tested is shown by way of example. Besides, there are the other sensors for respectively detecting breathing rate, body temperature, blood flow, and the like.

The pulse detected by the pulse sensor 101 is converted into digital data of pulse rate for one minute by a pulse sensor processing circuit 102.

Power is supplied from a battery 110 to the pulse sensor 101 and the pulse sensor processing circuit 102 via a constant voltage circuit 111 for keeping the voltage constant and a first power on/off circuit 103. The first power on/off circuit 103 can turn on and off the power of the pulse sensor 101 and the pulse sensor processing circuit 102, according to a control of a CPU 112. Thus, the power of the portion concerned with the pulse sensor can be cut off when detecting no pulse.

The acceleration detected by the acceleration sensor 104 is converted into digital data of acceleration by the acceleration sensor processing circuit 105.

The power is supplied from the battery 110 to the acceleration sensor 104 and the acceleration sensor processing circuit 105 via the constant voltage circuit 111 for keeping the voltage constant and a second power on/off circuit 106. According to a control of the CPU 112, the second power on/off circuit 106 can turn on/off the power of the acceleration sensor 104 and the acceleration processing circuit 105. Thus, the power of the portion concerned with the acceleration sensor can be cut off when detecting no acceleration.

The above sensor data is temporarily stored in a memory 114 according to a control of the CPU 112 controlling the whole measurement device in accordance with a program stored in a ROM 113. The sensor data temporarily stored in the memory 114 is transmitted by wireless by using a wireless transmitter/receiver 107 and an antenna 108. An instruction command for the measurement device is received by using the wireless transmitter/receiver 107 and the antenna 108.

The power is supplied from the battery 110 to the wireless transmitter/receiver 107 via the constant voltage circuit 111 for keeping the voltage constant and a third power on/off circuit 109. The third power on/off circuit 109 can turn on/off each power of a transmitter and a receiver, of the power supplied to the wireless transmitter/receiver 107, according to a control of the CPU 112.

Thus, the power of the wireless transmitter/receiver can be cut off when transmitting or receiving no data.

The current time can be called for from timer 115 by the CPU 112 and it can be used for storing the data sampling time of each sensor together with the sampling data or for judgment about transition to an intermittent receiving operation and the like described later.

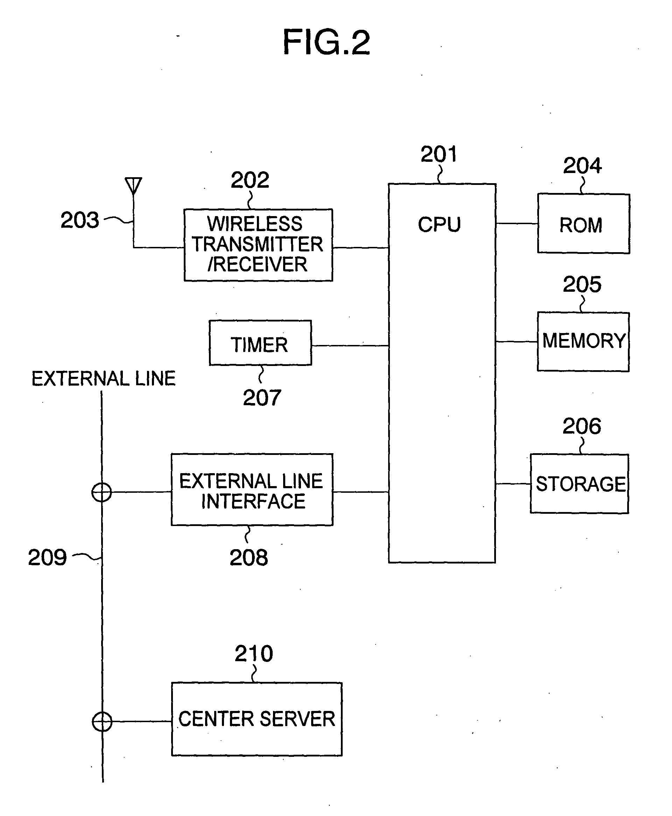

Fig. 2 is a view showing the hardware structure of a data collecting device according to one embodiment of the invention. The data collecting device is controlled by a CPU 201 operating according to a program stored in a ROM 204. It transmits an instruction command to the measurement device or receives the data transmitted from the measurement device, by using a wireless transmitter/receiver 202 and an antenna 203. The data received from the measurement device is temporarily stored in a memory 205 once and then stored in a storage 206. A hard disk and the like is used as the storage 206.

The current time can be read out from timer 207 by the CPU 201 and it is used for temporal synchronization with wireless communication, and the like.

In order to transfer the collected data further outward, the data collecting device is provided with an external line interface 208, which is connected to an external line 209. The external line includes a LAN, a public circuit, and the like and it is connected to a center server 210 via the above line. The center server puts the sensor data and the like from the data collecting device together, so to perform various analysis and services for the external.

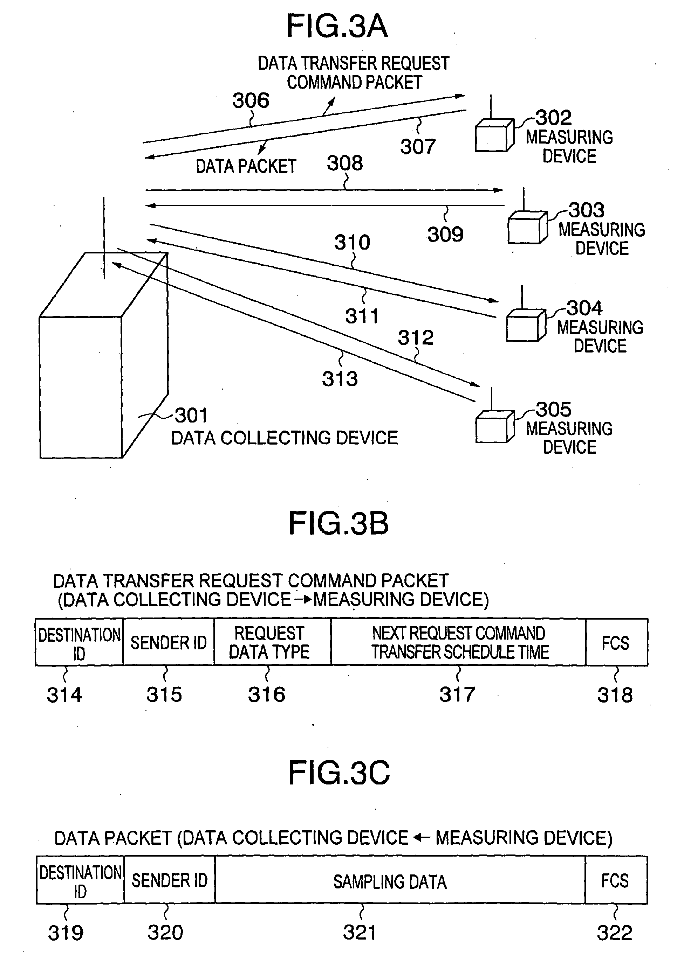

Fig. 3(A) is a view showing the outline of wireless transmission/reception between the data collecting device and the measurement devices. The data collecting device 301 collects data from a plurality of measuring devices 302, 303, 304, and 305. Here, it shows the case of calling for data from the respective measuring devices in the order of the measuring device 302, the measuring device 303, the measuring device 304, and the measuring device 305, at regular intervals of time.

At first, the data collecting device 301 transfers a data transfer request command packet 306 to the measuring device 302. Thereafter, the measuring device 302 returns the requested data packet 307.

Next, the data colleting device 301 transfers a data transfer request command packet 308 to the measuring device 303. Thereafter, the measuring device 303 returns the requested data packet 309.

Next, the data collecting device 301 transfers a data transfer request command packet 310 to the measuring device 304. Thereafter, the measuring device 304 returns the requested data packet 311.

Next, the data collecting device 301 transfers a data transfer request command packet 312 to the measuring device 305. Thereafter, the measuring device 305 returns the requested data packet 313.

A series of the above operations will be repeated. Actually, since the temporal intervals for calling for the data of the measuring device varies depending on the characteristics of the respective measuring devices, the respective data transfer request command packets are transferred to the respective measuring devices at the respective temporal intervals for calling for the data of the respective measuring devices and the respective data packets corresponding to the above command packets are transferred to the data collecting device.

Fig. 3(B) is a view showing the structure of the data transfer request command packet (306, 308, 310, 312) transferred from the data collecting device to the measuring device. The structure of the data transfer request command packet includes a destination ID 314, a sender ID 315, a request data type 316, a next request command transfer schedule time 317, and an FCS 318.

The destination ID 314 is the ID of a measuring device requested to transfer data and each of the measuring devices has the ID (number of two bytes and the like) for identification.

The sender ID 315 is the ID of the data collecting device.

The request data type 316 is the number for identifying the type of the data the data collecting device requests the measuring device to transfer, and for example, "0" is assigned to the pulse rate and "1" is assigned to the acceleration speed.

The next request command transfer schedule time 317 is the "transfer schedule time of the next data transfer request command" to the measuring device after the transmission of this data transfer request command packet. The data collecting device will not transfer the next data transfer request command to the measuring device until the schedule time. The measuring device receiving the data transfer request command packet turns off the power of the receiver of the wireless transmitter/receiver 107 until the transfer schedule time of the next data transfer request command, after the requested data transfer.

The FCS 318 is a code for detecting an error of a packet, and a CRC code of 16 bits which is calculated from the head of a packet to the last of the next request command transfer schedule time 317, and the like is used.

Fig. 3(c) is a view showing the structure of the data packet (307, 309, 311, 313) transferred from the measuring device to the data collecting device. The data packet includes a destination ID 319, a sender ID 320, sampling data 321, and an FCS 322.

The destination ID 319 is the ID of a data collecting device requesting the data transfer.

The sender ID 320 is the ID of a measuring device transferring the data packet.

The sampling data 321 is the data requested by the request data type 316 of the data transfer request command packet.

The FCS 322 is a code for detecting an error of a packet and the CRC code of 16 bits which is calculated the head of a packet to the last of the sampling data 321, and the like is used.

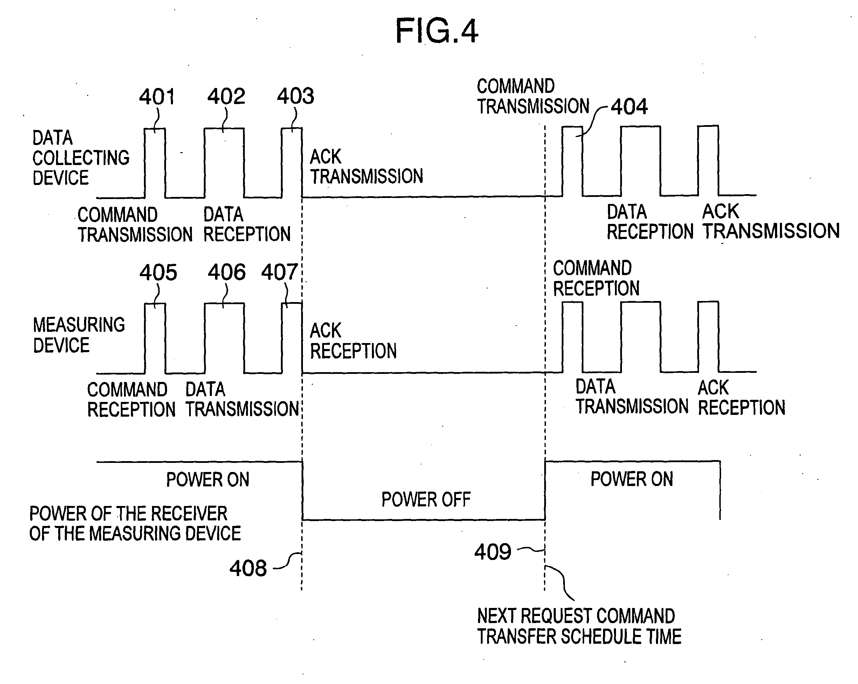

Fig. 4 is a timing chart of wireless transmission/reception between the data collecting device and the measuring device and power control of the receiver of the wireless transmitter/receiver 107 of the measuring device. A data transfer request command packet is transmitted from the data collecting device and received by the measuring device (401, 405). A data packet is transmitted from the measuring device and received by the data collecting device (406, 402). The data collecting device checks whether the received data packet is correct by using the FCS and the like, and when it is correct, it transfers an ACK packet. The measuring device receives the ACK packet, hence to complete a series of communication (403, 407). The ACK packet is a packet to be returned to the measuring device when the data collecting device has received the correct data packet, using a code of one byte. The measuring device cuts off the power of the receiver after receiving the ACK packet (timing of 408).

The measuring device turns on the power of the receiver (timing of 409) at the next request command transfer schedule time (317 of Fig. 3(B)) of the data transfer request command packet. The data collecting device transmits the next data transfer request command packet 404 at a time later than the next request command transfer schedule time (timing of 409).

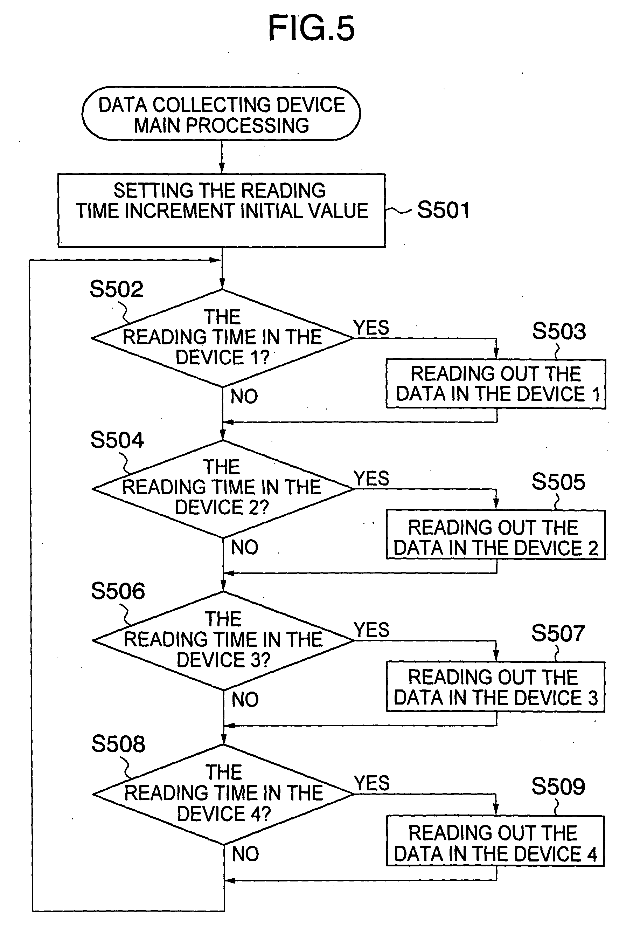

Fig. 5 is a flow chart showing the processing of the data collecting device. Here, it shows the case of reading out the sampling data from four measuring devices from the measuring device 1 to the measuring device 4.

In Step S501, the initial value of the data reading time increment is set in every measuring device. The data reading time increment means a difference in time from a transfer of the data transfer request command packet to the next transfer of the data transfer request command packet to a measuring device.

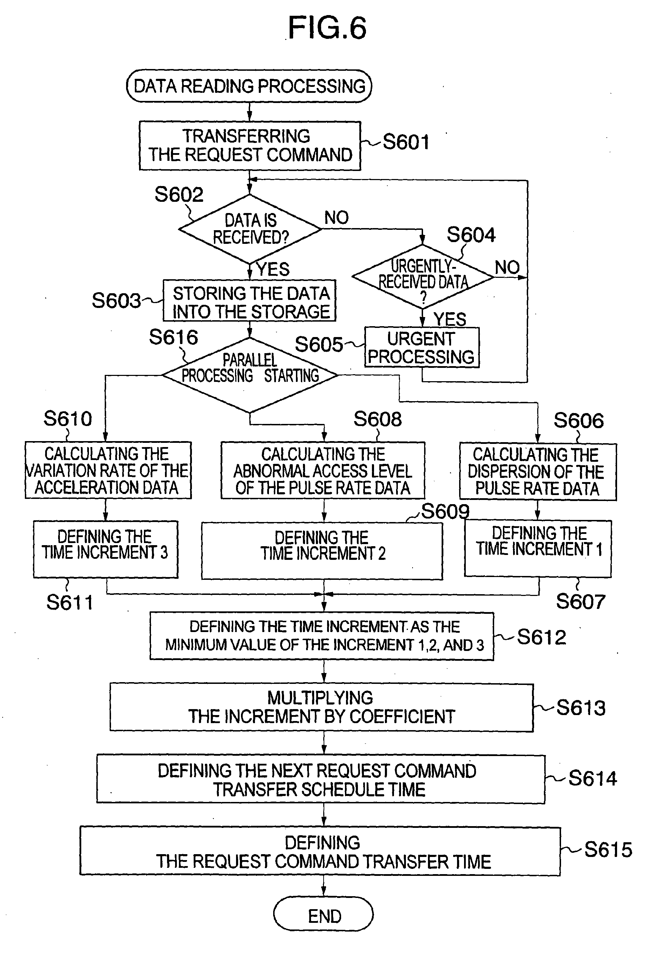

In Step S502, it is checked whether it becomes the data reading time from the measuring device 1. When it becomes the data reading time, the step proceeds to Step S503, where the data reading processing of the measuring device 1 is performed. The data reading processing of the measuring device will be described in Fig. 6.

In Step S504, it is checked whether it becomes the data reading time from the measuring device 2. When it becomes the data reading time, the step proceeds to Step S505, where the data reading processing of the measuring device 2 is performed.

In Step S506, it is checked whether it becomes the data reading time from the measuring device 3. When it becomes the data reading time, the step proceeds to Step S507, where the data reading processing of the measuring device 3 is performed.

In Step S508, it is checked whether it becomes the data reading time from the measuring device 4. When it becomes the data reading time, the step proceeds to Step S509, where the data reading processing of the measuring device 4 is performed.

The step returns to Step S502, where a series of the processing is repeated. When the number of the measuring devices is increased, the same processing from Step S508 and Step S509 can be added after Step S508, for the additional measuring device, hence to cope with the above situation.

Fig. 6 is a flow chart showing the details of the processing of reading data from the measuring device by the collecting device(Steps S503, S505, S507, and S509 in Fig. 5).

In Step S601, a data transfer request command packet is transmitted to a target measuring device. The time obtained by adding the reading time increment initial value set in Step S501 in Fig. 5 to the current time is first used as the "transfer schedule time of the next data transfer request command" 317 of the data transfer request command packet. Thereafter, the time calculated in Step S614 will be used.

In Step S602, it is checked whether the data packet has been received from the measuring device to which the data transfer request command packet has been transmitted. When the data packet has not been received, the step proceeds to Step S604, where it is checked whether there is the urgently-received data packet. The urgently-received data packet is a packet to be transmitted from the measuring device to the data collecting device at any timing when various sensor data detected by the measuring device becomes abnormal. When there is the urgently-received data packet in Step S604, the step proceeds to Step S605, where the urgent processing such as sounding the alarm or alerting the outside by using the LAN 209, is performed, and the step moves to the waiting processing of receiving a data packet in Step S602 again.

When the data packet has been received in Step S602, the step proceeds to Step S603, where the received data is stored in the storage 206. Since the data packet transmitted from the measuring device includes the detection time as well as the data detected by a sensor, the detection time is also stored in the storage 206 together with the data. Next, the step proceeds to Step S616, and Step S616 proceeds to Step S606, Step S608, or Step S610, respectively depending on the type of the living body information sensor.

In Step S606, the dispersion of the pulse rate data stored in the storage 206 within the time as far back as a predetermined period from the past to the present is calculated. The dispersion is obtained by calculating the standard deviation and the like.

In Step S607, the reading time increment 1 is defined by subtracting the product of the dispersion and a predetermined proportional multiplier J from the reading time increment initial value set in Step S501 in Fig 5. Accordingly, when the dispersion is large, the reading time increment 1 is decreased, while when the dispersion is small, the reading time increment 1 is increased.

In Step S608, the abnormal access level of the pulse rate data stored in the storage 206 within the time as far back as a predetermined period from the past to the present, is calculated. For the abnormal access level, the absolute value and the like of the value obtained by subtracting the medium value between the predetermined upper limit of the pulse rate and lower limit of the pulse rate from the average value of the pulse rate is adopted.

In Step S609, the reading time increment 2 is defined by the value of subtracting the product of (1/abnormal access level) and a predetermined proportional multiplier K from the reading time increment initial value set in Step S501 of Fig. 5. Accordingly, when the abnormal access level is large, the reading time increment 2 is decreased, while when the abnormal access level is small, the reading time increment 2 is increased.

In Step S610, the variation rate of the acceleration data stored in the storage 206 within the time as far back as a predetermined period from the past to the present is calculated. For the variation rate of acceleration, a difference and the like between the maximum acceleration and the minimum acceleration within the above period of time is adopted.

In Step S611, the reading time increment 3 is defined by the value of subtracting the product of the variation rate of acceleration and a predetermined proportional multiplier L from the reading time increment initial value set in Step S501 of Fig. 5. Accordingly, when the variation rate of acceleration is large, the reading time increment 3 is decreased, while when the variation rate of acceleration is small, the reading time increment 3 is increased.

In Step S612, the minimum one of the reading time increment 1, the reading time increment 2, and the reading time increment 3 is defined as the reading time increment used in the steps thereafter.

In Step S613, the reading time increment is defined by multiplying the reading time increment obtained in Step S612 by the time correction coefficient. The value of the time correction coefficient is obtained by analyzing the temporal tendency of the data collected by the data collecting device with the analytical software operating in the CPU 201. For example, it is set at a small value during the daytime when a large change occurs in the sensor detection data and at a large value during the night when little change occurs in the sensor detection data.

In Step S614, the "transfer schedule time of the next data transfer request command" (317 in Fig. 3(B)) specified in the data transfer request command packet is defined by the value obtained by adding the reading time increment calculated in Step S613 to the current time read from the timer 207.

In Step S615, the time to transfer the data transfer request command packet to the measuring device actually is defined by the value obtained by adding a predetermined time allowance C to the "transfer schedule time of the next data transfer request command" calculated in Step S614.

As mentioned above, although the case of using the pulse sensor and the acceleration sensor as the sensor of the measuring device has been shown, the processing for the data dispersion (S606, S607), the processing for the data abnormal access level (S608, S609), and the processing for the data variation rate (S610, S611) can apply to the other sensors (breathing rate, body temperature, blood flow, and the like).

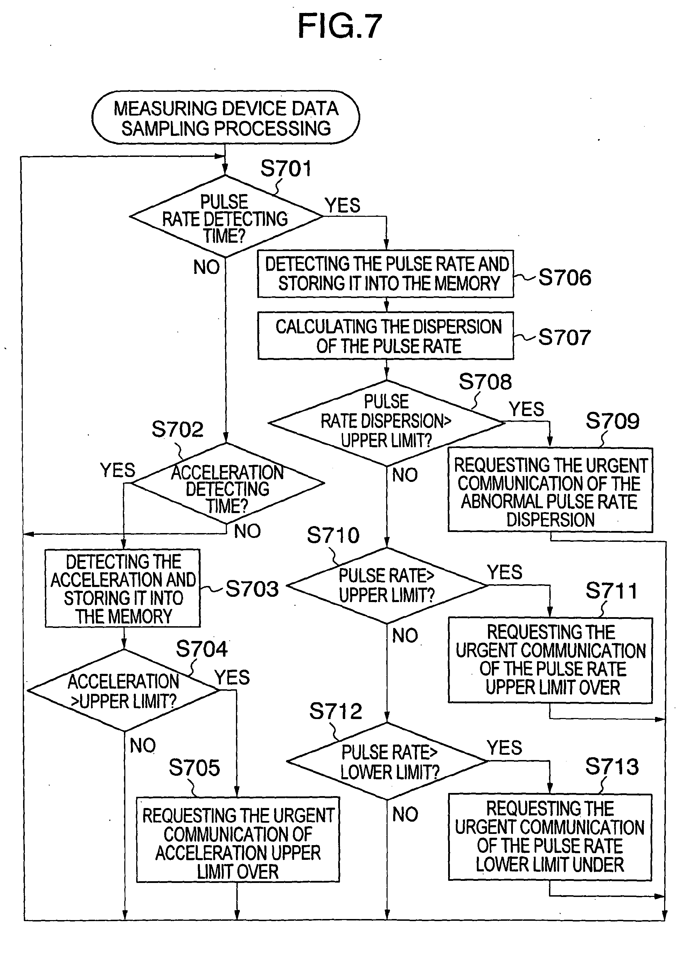

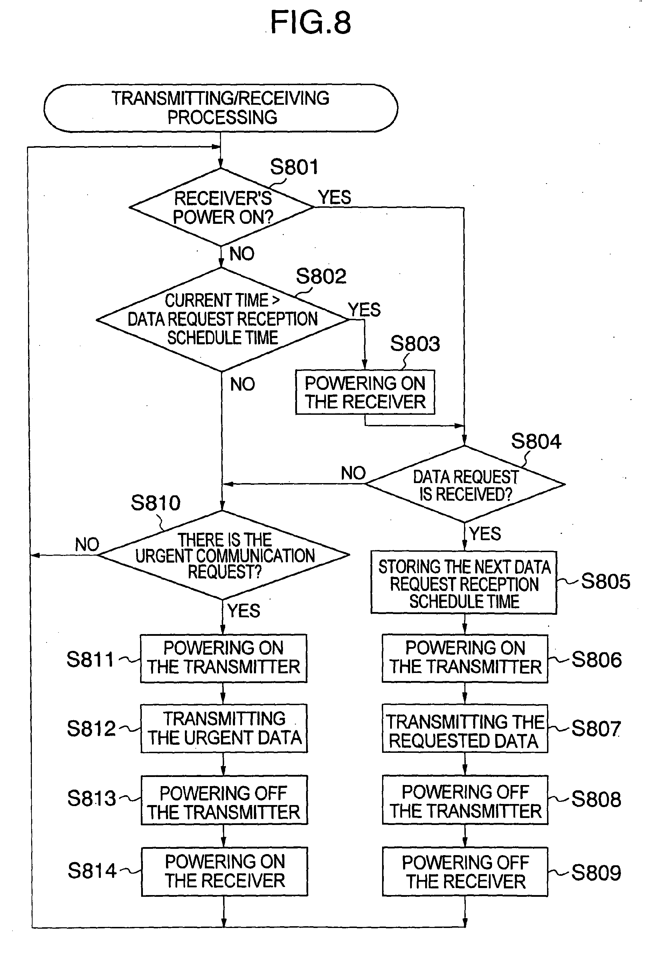

Fig. 7 and Fig. 8 are flow charts showing the processing of the measuring device. Fig. 7 shows the processing of sampling data from a sensor, and Fig. 8 shows the processing of transmitting and receiving data by wireless. The data sampling processing of Fig. 7 and the data transmission/reception processing of Fig. 8 are simultaneously performed according to the operation system run by the CPU 112. Although the case of using the pulse sensor and the acceleration sensor as the sensor of the measuring device has been shown, the same algorithm can apply to the other sensors (breathing rate, body temperature, blood flow, and the like).

In Step S701 of Fig. 7, it is checked whether it is the pulse rate detection time or not. When it is the pulse rate detection time, the step proceeds to Step S706, and otherwise, it proceeds to Step 702. The pulse rate is read out from the pulse sensor (101 in Fig. 1) via pulse rate calculating means (102 in Fig. 1) in Step S706 and it is stored in the memory (114 in Fig. 1) together with the current time read from the timer (115 in Fig. 1).

The pulse rate dispersion within the predetermined time period, of the pulse rate so far stored in the memory 114, is calculated in Step S707. The pulse rate dispersion is obtained by calculating the standard deviation and the like.

In Step S708, it is checked whether the pulse rate dispersion exceeds the upper limit or not. When it exceeds the upper limit, the step proceeds to Step S709, where the urgent communication for pulse rate abnormal dispersion is required in the data transmission/reception processing described in Fig. 8. When it does not exceed the upper limit, the step proceeds to Step 710.

In Step S710, it is checked whether the pulse rate exceeds the upper limit or not. When it exceeds the upper limit, the step proceeds to Step S711, where the urgent communication for the pulse rate over-limit is required in the data transmission/reception processing described in Fig. 8. When it does not exceed the upper limit, the step proceeds to Step 712.

In Step 712, it is checked whether the pulse rate is below the lower limit or not is checked. When it is below the lower limit, the step proceeds to Step S713, where the urgent communication for the pulse rate underlimit is required in the data transmission/reception processing described in Fig. 8.

In Step S702, it is checked whether it is the detection time of acceleration. The acceleration is detected at regular intervals. When it is the detection time of acceleration, the step proceeds to Step S703. The acceleration is read out from the acceleration sensor (104 in Fig. 1) via acceleration processing means (105 in Fig. 1) in S703, and it is stored in the memory (114 in Fig. 1) together with the current time read from the timer (115 in Fig. 1).

It is checked whether the acceleration detected in Step S704 exceeds the upper limit. When it exceeds the upper limit, the step proceeds to Step S705, where the urgent communication for the acceleration over-limit is required in the data transmission/reception processing described in Fig. 8.

Fig. 8 is a flow chart showing the data transmission/reception processing of the measuring device.

In Step S801, it is checked whether the receiver of the wireless transmitter/receiver is on the power. When it is on the power, since the measuring device is in a state of waiting for a command from the data collecting device, the step proceeds to Step S804, where the reception of the data transfer request command packet is checked. Otherwise, the step proceeds to Step S802.

In Step S802, it is checked whether the current time exceeds the reception schedule time of the data transfer request command. For the reception schedule time of the data transfer request command, the value of the "transfer schedule time of the next data transfer request command" (317 in Fig. 3(B)) of the data transfer request command packet having been received last time is adopted. When the current time exceeds the reception schedule time of the data transfer request command in Step S802, the step proceeds to Step S803, where the receiver of the wireless transmitter/receiver (107 in Fig. 1) is turned on, and then it proceeds to Step S804, where it waits for receipt of the data transfer request command packet from the data collecting device.

When the current time does not pass the reception schedule time of the data transfer request command in Step S802, the step proceeds to Step S810.

When the data transfer request command packet has been received in Step S804, the step proceeds to Step S805, where the value of the "transfer schedule time of the next data transfer request command" (317 in Fig. 3(B)) of the received data transfer request command packet is stored. When the data transfer request command packet has not been received, the step proceeds to Step S810.

In Step S806, the transmitter of the wireless transmitter/receiver is turned on, and in Step S807, the data packet specified by the "request data type" (316 in Fig. 3(B)) of the data transfer request command packet is transmitted. In Step S808, the transmitter of the wireless transmitter/receiver is turned off, and in Step S809, the receiver of the wireless transmitter/receiver is turned off.

In Step S810, it is checked whether there is the urgent communication request from the data sampling processing described in Fig. 7. When there is no urgent communication request, the step returns to Step S801. When there is the urgent communication request, the step proceeds to Step S811.

In Step S811, the transmitter of the wireless transmitter/receiver is turned on. In Step S812, the urgent communication data requested from the data sampling processing of Fig. 7 is transmitted. In Step S813, the transmitter of the wireless transmitter/receiver is turned off. In Step S814, the receiver is turned on because there is a possibility of receiving a command packet from the data collecting device as for the urgent communication data having transmitted in Step S812, the step returns to Step S801, and in Step S804, it waits for a command from the data collecting device.

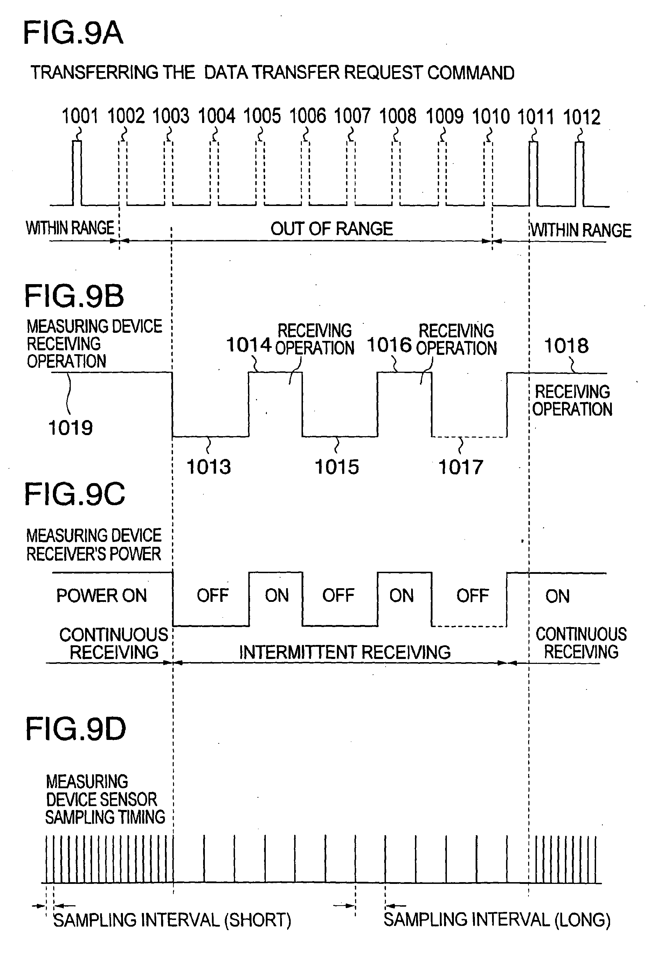

Fig. 9 is a timing chart showing a transition between continuous reception and intermittent reception in the receiver and a change in the sampling intervals of the sensor, when the measuring device moves out of range of the radio wave from the data collecting device.

Fig. 9(A) is a timing chart of transmission of the data transfer request command from the data collecting device to the measuring device, in which 1001 to 1012 indicate the data transfer request commands respectively. Here, the data transfer request commands 1001, 1011, and 1012 shown by the solid line are the data transfer request commands transmitted when the measuring device is within range of the radio wave from the data collecting device, while 1002 to 1010 shown by the dotted line are the data transfer request commands transmitted when the measuring device is out of range of the radio wave from the data collecting device.

Fig. 9(B), (C) is a view showing the relationship between the receiving operation of the measuring device and the power on/off of the receiver. In the state of 1012 in Fig. 9(B), although it shows the continuous receiving operation, the receiver moves to the intermittent receiving operation when it cannot receive the data transfer request command from the data collecting device for a predetermined period of time.

During the intermittent receiving operation, the receiver is intermittently turned on, so to perform the receiving operation for a predetermined period of time, as shown in the state from 1013 to 1017 in Fig. 9(B). In the state of 1013, the receiver is turned off for the predetermined period of time, and in the state of 1014, the receiver is turned on, so to perform the receiving operation for the predetermined period of time. In the state of 1015, the receiver is again turned off for the predetermined period of time, and in the state of 1016, the receiver is turned on, so to perform the receiving operation for the predetermined period of time. The above operations will be repeated in the state of 1017 and the later.

When it can receive the data transfer request command from the data collecting device at the receiving time during the intermittent receiving operation, the operation turns to the continuous receiving operation (state of 1018).

Fig. 9(D) is a view showing the timing of the data sampling from the sensor of the measuring device. The receiver shifts to the intermittent receiving operation because the measuring device receives no data transfer request command from the data collecting device for the predetermined period of time, and simultaneously, the sampling intervals of the sensor are lengthened. Here, in an interval between one sampling and another sampling of the sensor, the sensor and its related part are turned off and the electric power consumed in the sensor and its related part is saved and the memory consumption for storing the data detected by the sensor is saved.

When it receives the data transfer request command from the data collecting device again during the intermittent receiving operation of the receiver, the sampling intervals of the sensor is returned to the ordinary intervals and the corresponding data including the data sampled during the receiver's intermittent receiving operation is transmitted to the data collecting device.

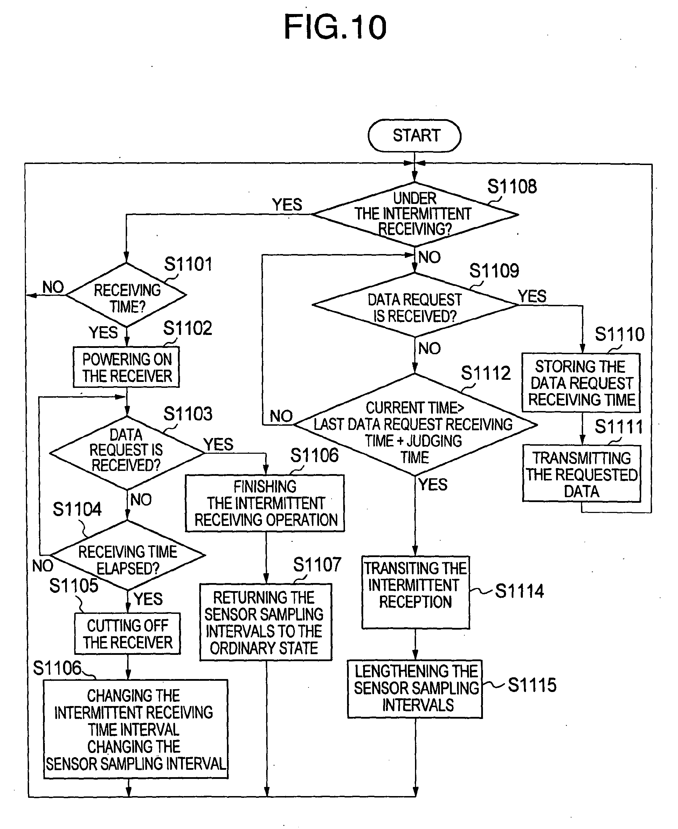

Fig. 10 is a flow chart showing the judgment processing of transition to the intermittent receiving operation of the measuring device.

In Step S1108, the receiver judges whether it is under the intermittent receiving operation. The transition to the intermittent receiving operation is performed at the last execution in Step S1114 described later. When it is not under the intermittent receiving operation, the step proceeds to Step S1109, where it is judged whether the data transfer request command from the data collecting device is received or not. When the data transfer request command is received, the step proceeds to Step S1110, where the time of receiving the data transfer request command is stored into the memory (114 in Fig. 1), and then, proceeding to Step S1111, the data requested by the data transfer request command is transmitted to the data collecting device. When there is the data sampled from the sensor during the intermittent reception described later, the same data is also transmitted there.

When it could not receive the data transfer request command from the data collecting device in Step S1109, the step proceeds to Step S1112, where it is checked whether the current time exceeds the time obtained by adding the predetermined judgment time to the last receiving time of the data transfer request command. This judgment time is the time for turning the receiver to the intermittent receiving operation when it fails to receive the data transfer request command from the data collecting device for the predetermined period of time. This step S1112 is the judging means of operation change to the intermittent receiving operation.

When the current time does not exceed the time obtained by adding the judgment time to the last receiving time of the data transfer request command, the step returns to Step S1109, where the reception of the data transfer request command from the data collecting device is again checked.

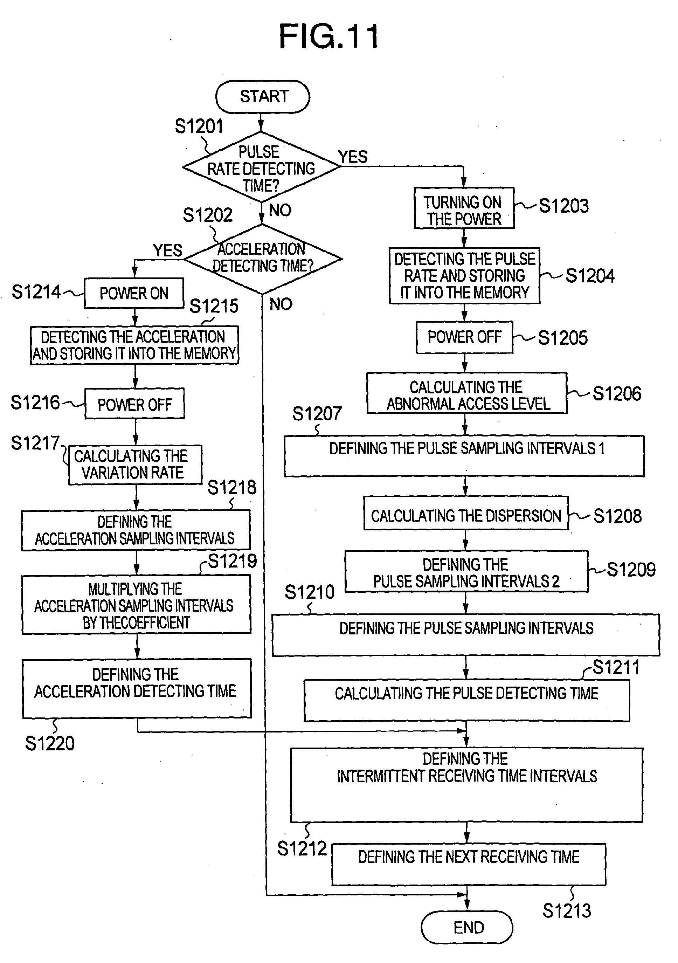

When the current time exceeds the time obtained by adding the judgment time to the last receiving time of the data transfer request command, it is judged that the measuring device moves out of range of the radio wave of the data collecting device, and therefore, in order to save the electric power of the receiver, the processing for turning the receiver to the intermittent receiving operation is performed in Step S1114. Since the data sampled by the sensor cannot be transmitted to the data collecting device, the sampling intervals are lengthened in Step S1115, the number of the sampling data per unit of time is lessened, and the storing amount of the sampling data into the memory is decreased, hence to prevent from overflow of the memory. In the actual processing here, a reference value of the sampling intervals described in Fig. 11 is changed to a predetermined reference value of the sampling intervals under the intermittent receiving operation. The reference value under the intermittent receiving operation is fixed in advance at a larger value than the reference value of the continuous receiving operation.

When it is judged that the receiver is under the intermittent receiving operation in Step S1108, the step proceeds to Step S1101. The case where it is under the intermittent receiving operation means the case where it cannot receive the data transfer request command from the data collecting device for the predetermined time period and more, and this is checked in the above step S1112 and the shifting processing is performed in Step S1114.

In Step S1101, it is checked whether it is the time of receiving the radio wave from the data collecting device. The time of receiving is decided in Step S1213 of Fig. 11 described later. At the intermittent receiving operation of the receiver, generally the receiver is turned off and at the regular intervals, the receiver is turned on, so to perform the receiving operation for the predetermined period of time.

When it is judged that it is the time of receiving the radio wave from the data collecting device in Step S1101, the step proceeds to Step S1102, where the receiver is turned on while controlling the third power on/off circuit 109 of Fig. 1, then proceeding to Step S1103, it is checked whether the data transfer request command from the data collecting device is received or not. When it is judged that it is not the time of receiving the radio wave from the data collecting device, the step proceeds to Step S1108.

In Step S1103, when it can receive the data transfer request command, since it can be judged that the receiver is within range of the radio wave from the data collecting device, the finishing processing of the intermittent receiving operation of the receiver is performed in Step S1106, and the sampling intervals of the sensor is returned to the ordinary intervals in Step S1107, hence to shift to the ordinary operation. This step S1103 is the operation return judging means toward the ordinary operation.

When it cannot receive the data transfer request command in Step S1103, it is checked whether the predetermined receiving time has elapsed or not in Step S1104.

When the predetermined receiving time does not have elapsed in Step S1104, the step returns to Step S1103, where the reception about the data transfer request command is checked again.

When the predetermined receiving time has elapsed in Step S1104, the step proceeds to Step S1105, where the receiving operation is finished and the receiver is turned off until the next receiving time.

In Step S1116, the changing processing of the intermittent receiving time intervals and the sensor sampling intervals is performed according to the sensor detection data (described in Fig. 11).

Fig. 11 is a flow chart showing the details of the processing of changing the intermittent receiving interval and the processing of changing the sensor sampling interval in Step S1116 of Fig. 10.

In Step S1201, it is checked whether it is the detection time of the pulse rate. When it is the detection time of the pulse rate, the step proceeds to Step S1203. The detection time of the pulse rate is determined by the last execution of Step S1211 described later. When it is not the detection time of the pulse rate, the step proceeds to Step S1202.

In Step S1203, the pulse sensor and its related part are turned on. The power is turned on by the CPU 112 controlling the first power on/off circuit 103 in Fig. 1.

In Step S1204, the pulse rate is detected and it is stored in the memory (114 in Fig. 1) together with the time, and in Step S1205, the pulse sensor and its related part are turned off.

In Step S1206, the abnormal access level of the data within the time as far back as a predetermined period from the past to the present, of the pulse rate data stored in the memory (114 in Fig. 1), is calculated. For the abnormal access level, the absolute value of the value obtained by subtracting the medium value between the predetermined upper limit of the pulse rate and lower limit of the pulse rate from the average value of the pulse rate and the like is adopted.

In Step S1207, the pulse sampling interval 1 is defined by the value obtained by subtracting the product of (1/anbormal access degree) and the predetermined proportional multiplier K from the reference value of the pulse sampling interval 1 previously set. Thus, when the abnormal access level is large, the pulse sampling interval 1 becomes small, while when the abnormal access level is small, the pulse sampling interval 1 becomes large.

In Step S1208, the dispersion of the data within the time as far back as a predetermined period from the past to the present, of the pulse rate data stored in the memory (114 in Fig. 1), is calculated. The dispersion is obtained by calculating the standard deviation and the like.

In Step S1209, the pulse sampling interval 2 is defined by the value obtained by subtracting the product of the dispersion and the predetermined proportional multiplier J from the predetermined reference value of the pulse sampling interval 2. Thus, when the dispersion is large, the pulse sampling interval 2 becomes small, while when the dispersion is small, the pulse sampling interval 2 becomes large.

In Step S1210, the product obtained by multiplying the smaller one of the pulse sampling interval 1 required in Step S1207 and the pulse sampling interval 2 required in Step S1209 by time correction coefficient is defined as the pulse rate sampling interval. The value of the time correction coefficient is obtained by analyzing the tendency of the data detected by the pulse sensor in a period of time. For example, it is set at a small value during the daytime when a large change occurs in the pulse sensor detection data and at a large value during the night when little change occurs in the pulse sensor detection data.

In Step S1211, the next pulse rate detection time is calculated by adding the pulse rate sampling interval required in Step S1210 to the current time. This pulse rate detection time is used for judgment at the next execution of Step S1201.

In Step S1202, it is checked whether it is the detection time of acceleration or not. When it is the detection time of acceleration, the step proceeds to Step S1214. The detection time of acceleration is determined by the last execution of Step S1220 described later. When it is not the detection time of acceleration, the processing of Fig. 11 is finished.

In Step S1214, the acceleration sensor and its related part are turned on. The power is turned on by the CPU 112 controlling the second power on/off circuit 106 of Fig. 1.

In Step S1215, the acceleration is detected and it is stored in the memory (114 in Fig. 1) together with the time, and in Step S1216, the acceleration sensor and its related part are turned off.

In Step S1217, the variation rate of acceleration of the data within the time as far back as a predetermined period from the past to the present, of the acceleration data stored in the memory (114 in Fig. 1), is calculated. For the variation rate of acceleration, a difference between the maximum acceleration and the minimum acceleration and the like within the period of time, is adopted.

In Step S1218, the acceleration sampling interval is defined by the value obtained by subtracting the product of the variation rate of acceleration and the predetermined proportional multiplier L from the predetermined reference value of acceleration sampling intervals. Thus, when the variation rate of acceleration is large, the acceleration sampling intervals become small, while when the variation rate of acceleration is small, the acceleration sampling intervals become large.

In Step S1219, the product obtained by multiplying the acceleration sampling intervals required in Step S1218 by the time correction coefficient is defined as the acceleration sampling intervals. The value of the time correction coefficient is obtained by analyzing the tendency of the data detected by the acceleration sensor in a period of time. For example, it is set at a small value during the daytime when a large change occurs in the acceleration sensor detection data and at a large value during the night when only a little change occurs in the acceleration sensor detection data.

In Step S1220, the next acceleration detection time is obtained by adding the acceleration sampling intervals required in Step S1219 to the current time. The obtained acceleration detection time is used for judgment at the next execution of Step S1202.

In Step S1212, the intermittent receiving time intervals of the receiver are defined by the product obtained by multiplying all additions of the product of the pulse sampling interval 1 and the proportional constant A, the product of the pulse sampling interval 2 and the proportional constant B, and the product of the acceleration sampling interval and the proportional constant C, by the proportional multiplier D. The proportional multipliers A, B, C, and D are predetermined.

In Step S1213, the next receiving time is defined by adding the intermittent receiving time intervals required in Step S1212 to the current time. The receiving time value is used for judgment at the next execution of Step S1101 of Fig. 10.

As mentioned above, although the case of using the pulse sensor and the acceleration sensor as the sensor of the measuring device has been shown, the processing for the abnormal access level of the data detected by the sensor (S1206, S1207), the processing for the data dispersion (S1208, S1209), the processing for the variation rate of data (S1217, S1218), and the processing concerned with the time correction (S1210, S1219) and the like can apply to the other sensors (breathing rate, body temperature, blood flow, and the like).

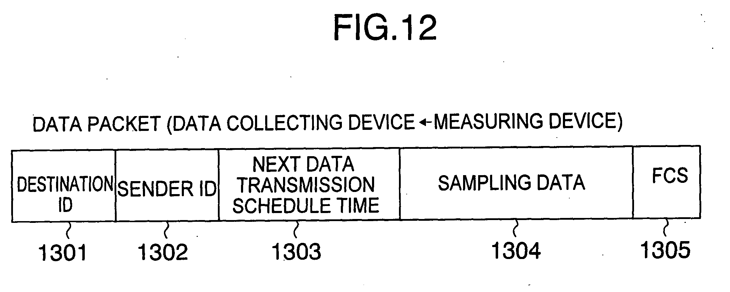

Fig. 12 is a view showing the structure of the data packet in the case of installing a function of calculating the "next data transmission schedule time" in the measuring device. In the case of this installation, the schedule time value corresponding to the above "transfer schedule time of the next data transfer request command" is calculated on the side of the measuring device and transmitted to the data collecting device.

The data collecting device determines the "transfer schedule time of the next data transfer request command" based on the "next data transmission schedule time" sent from the measuring device.

The data packet includes the destination ID 1301, the sender ID 1302, the next data transmission schedule time 1303, the sampling data 1304, and the FCS 1305.

The destination ID 1301 is the ID of the data collecting device to which the measuring device transmits the data packet.

The sender ID 1302 is the ID of the measuring device which transfers the data packet.

The next data transmission schedule time 1303 is the "next data transmission schedule time", and after transmission of this data packet, the wireless transmitter/receiver (107 in Fig. 1) of the measuring device is turned off the power until the next transmission schedule time of the data packet, hence to save the power of the measuring device.

Since the wireless transmitter/receiver of the measuring device is turned on at about the "next data transmission schedule time" of the data packet sent from the measuring device, the data collecting device transmits the instruction information to the measuring device in accordance with the above time.

The sampling data 1304 is the data sampled by the measuring device for a period from the last data packet transmission to this data packet transmission.

The FCS 1305 is the code for detecting an error of a packet, and the CRC code of 16 bits which is calculated the head of a packet to the last of the sampling data 1304 and the like is adopted for the FCS 1305.

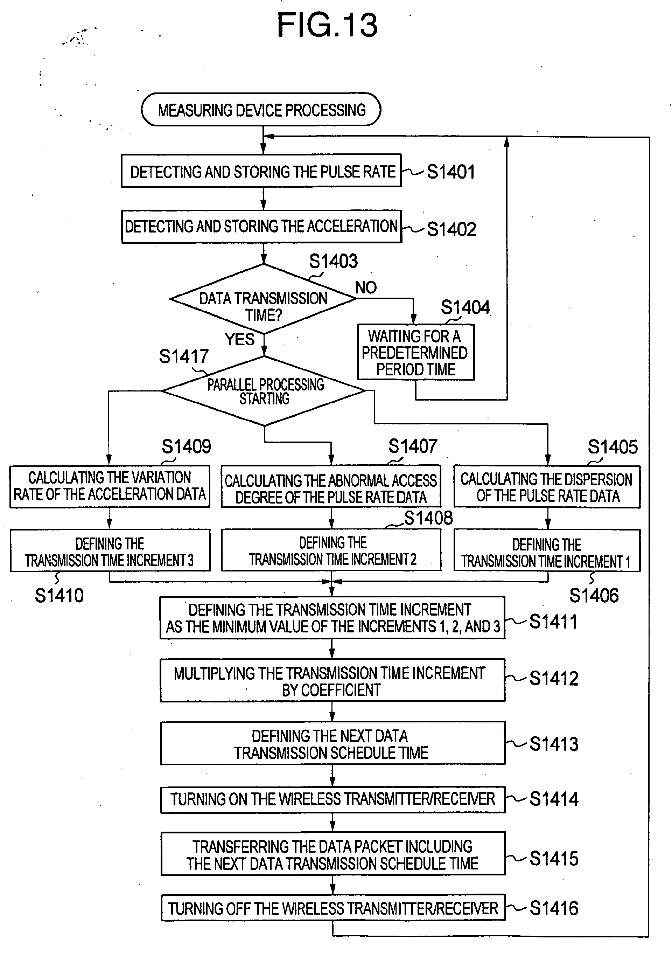

Fig. 13 is a flow chart showing the processing on the side of the measuring device when installing the function of calculating the "next data transmission schedule time" in the measuring device.

In Step S1401, the pulse rate is detected and stored into the memory (114 in Fig. 1).

In Step S1402, the acceleration is detected and stored into the memory (114 in Fig. 1).

In Step S1403, it is checked whether it is the time to transmit the data on the pulse rate and the acceleration stored in the memory to the data collecting device. For the data transmitting time in this case, the next data transmission schedule time calculated in the last execution of Step S1413 described later is adopted. When it is judged that it is not the data transmitting time in Step S1403, the step proceeds to Step S1404, and after waiting for a predetermined period, it returns to Step S1401 again. The predetermined period in Step S1404 means the sampling intervals for detecting the pulse rate and the acceleration.

When it is judged that it is the data transmitting time in Step S1403, the step proceeds to Step S1417, and from Step S1417, it further proceeds to Step S1405, Step S1407, and Step S1409, depending on the type of the living body information sensor.

In Step S1405, the dispersion of the data within the time as far back as a predetermined period from the past to the present, of the pulse rate data stored in the memory (114 in Fig. 1), is calculated. The dispersion is obtained by calculating the standard deviation and the like.

In Step S1406, the data transmitting time increment 1 is defined by the value obtained by subtracting the product of the dispersion and the predetermined proportional multiplier J from the predetermined transmitting time increment initial value Thus, when the dispersion is large, the data transmitting time increment 1 becomes small, and when the dispersion is small, the data transmitting time increment 1 becomes large.

In Step S1407, the abnormal access level of the data as far back as a predetermined period from the past to the present, of the pulse rate data stored in the memory (114 in Fig. 1), is calculated. For the abnormal access level, the absolute value of the value obtained by subtracting the medium value between the predetermined upper limit of the pulse rate and lower limit of the pulse rate from the average of the pulse rate and the like, is adopted.

In Step S1408, the data transmitting time increment 2 is defined by the value obtained by subtracting the product of (1/abnormal access level) and the predetermined proportional multiplier K from the transmitting time increment initial value previously set. Thus, when the abnormal access level is large, the data transmitting time increment 2 becomes small, while when the abnormal access level is small, the data transmitting time increment 2 becomes large.

In Step S1409, the variation rate of acceleration of the data within the time as far back as a predetermined period from the past to the present, of the acceleration data stored in the memory (114 in Fig. 1), is calculated. For the variation rate of acceleration, it adopts a difference between the maximum acceleration and the minimum acceleration in the above period of time, and the like.

In Step S1410, the data transmitting time increment 3 is defined by the value obtained by subtracting the product of the variation rate of acceleration and the predetermined proportional multiplier L from the transmitting time increment initial value previously set. Thus, when the variation rate of acceleration is large, the data transmitting time increment 3 becomes small, while when the variation rate of acceleration is small, the data transmitting time increment 3 becomes large.

In Step S1411, the smallest one of the data transmitting time increment 1, the data transmitting time increment 2, and the data transmitting time increment 3 is defined as the data transmitting time increment used for the following steps.

In Step S1412, the product obtained by multiplying the data transmitting time increment obtained in Step S1411 by the time correction coefficient is defined as the transmitting time increment. The value of the time correction coefficient is obtained by analyzing the tendency of the collected data in a period of time according to the analytic software running on the CPU (112 in Fig. 1). For example, it is set at a small value during the daytime when a large change occurs in the sensor detection data and at a large value during the night when only a small change occurs in the sensor detection data.

In Step S1413, the "next data transmission schedule time" specified in the data packet (1303 in Fig. 12) is defined by the value obtained by adding the transmitting time increment calculated in Step S1412 to the current time read out from the timer (115 in Fig. 1).

In Step S1414, the wireless transmitter/receiver (107 in Fig. 1) is turned on. In Step S1415, the data packet including the "next data transmission schedule time" and the sampling data is transferred to the data collecting device.

In Step S1416, the wireless transmitter/receiver (107 in Fig. 1) is turned off.

As mentioned above, although the case of using the pulse sensor and the acceleration sensor as the sensor of the measuring device has been shown, the processing for the data dispersion (S1405, S1406), the processing for the data abnormal access level (S1407, S1408), and the processing for the data variation rate (S1409, S1410) can apply to the other sensors (breathing rate, body temperature, blood flow, and the like).

## Claims

1. A living body information measuring system comprising
a measuring device including measuring means for measuring living body information and transmitting means for transmitting living body information data supplied from the measuring means based on the living body information, and
a data collecting device for receiving the living body information data from the measuring device, in which
the data collecting device transmits a data transfer request command including type of the living body information data and a first data transmission schedule time that is a schedule time for transmitting the next data of the living body information data.

2. The living body information measuring system according to Claim 1, wherein the measuring device cuts off power of a receiver after receiving the data transfer request command from the data collecting device.

3. The living body information measuring system according to Claim 2, wherein the measuring device cuts off the power of the receiver, and turns on the power of the receiver after elapse of the first data transmission schedule time.

4. The living body information measuring system according to Claim 3, wherein the data collecting device has a function of determining the first data transmission schedule time within the data transfer request command, based on a value of the living body information data received from the measuring device.

5. The living body information measuring system according to Claim 4, wherein the data collecting device has a function of setting the first data transmission schedule time within the data transfer request command at a larger value according as smaller is a difference between a medium value required from an upper limit and a lower limit previously set in a storage within the data collecting device and the value of the living body information data received from the measuring device.

6. The living body information measuring system according to Claim 4, wherein
the data collecting device has a function of setting the first data transmission schedule time within the data transfer request command at a larger value according as dispersion of the living body information data received from the measuring device is smaller, and
setting the first data transmission schedule time at a smaller value according as the dispersion is larger.

7. The living body information measuring system according to Claim 4, wherein
the data collecting device has a function of setting the first data transmission schedule time within the data transfer request command at a larger value according as a variation rate of the living body information data received from the measuring device is smaller, and
setting the first data transmission schedule time at a smaller value according as the variation rate is larger.

8. The living body information measuring system according to Claim 4, wherein the data collecting device has a function of determining the first data transmission schedule time within the data transfer request command, based on the time received from the measuring device.

9. The living body information measuring system according to Claim 4, wherein the measuring device transmits the living body information data to the data collecting device at arbitrary timing when the living body information measured by the measuring device is larger than a predetermined upper limit previously set in the storage within the measuring device or smaller than a lower limit.

10. The living body information measuring system according to Claim 3, wherein
when the measuring device does not receive any data transfer request command from the data collecting device for a predetermined period of time,
the measuring device includes operation change judging means for extending measuring sampling intervals for measuring the living body information, and
storing means for storing into the storage the living body information during a period of receiving none of the data transfer request command from the data collecting device.

11. The living body information measuring system according to Claim 3, wherein when the measuring device does not receive the data transfer request command from the data collecting device for a predetermined period of time, the measuring device performs an intermittent receiving operation that is regular repetition of power on/off of the receiver.

12. The living body information measuring system according to Claim 11, wherein
when the measuring device receives a data transfer request command from the data collecting device during the intermittent receiving operation,
the measuring device changes the receiver to a continuous receiving operation,
performs a judgment for turning the measuring sampling intervals of the living body information measuring means to an ordinary state, and
transmits the data stored in the storage together with the latest living body information to the data collecting device.

13. The living body information measuring system according to Claim 11, wherein the measuring device determines receiving intervals of the intermittent receiving period and the sampling intervals of the living body information sensor, based on the living body information measured by a living body information sensor within the measuring device, during the intermittent receiving operation.

14. The living body information measuring system according to Claim 13, wherein
according as the living body information measured by the living body information sensor approaches the predetermined upper limit or lower limit,
the measuring device sets the intermittent receiving time intervals and the sampling intervals at shorter periods than reference values of the intermittent receiving time, and
according as the living body information approaches the medium value of the upper limit and the lower limit, the measuring device sets the above intervals close to the reference values at the intermittent receiving time.

15. The living body measuring system according to Claim 13, wherein
according as the dispersion of the living body information measured by the living body information sensor is larger,
the measuring device sets the intermittent receiving time intervals and the sampling intervals of the living body sensor at shorter periods than the predetermined reference values at the intermittent receiving time, and
according as the dispersion of the living body information data is smaller, the measuring device sets the above intervals close to the reference values at the intermittent receiving time.

16. The living body measuring system according to Claim 13, wherein
according as the variation rate of the living body information measured by the living body information sensor is larger, the measuring device sets the intermittent receiving time intervals and the sampling intervals of the sensor at shorter periods than the predetermined reference values at the intermittent receiving time, and
according as the variation rate of the living body information data is smaller,
the measuring device sets the above intervals close to the reference values at the intermittent receiving time.

17. The living body information measuring system according to Claim 13, wherein the measuring device determines the intermittent receiving time intervals and the sampling intervals of the sensor based on the sampling time by the living body information sensor.

18. The living body information measuring system according to Claim 3, wherein the measuring device includes a second data transmission schedule time determined by the measuring device in response data to the data transfer request command.

19. The living body information measuring system according to Claim 18, wherein the data collecting device determines the data transmission schedule time based on the second data transmission schedule time and the measurement data transmitted from the measuring device.

20. The living body information measuring system according to Claim 19, wherein
the measuring device has a function of setting the second data transmission schedule time of the response data at a larger value according as smaller is a difference between the measurement data and the medium value obtained from the upper limit and the lower limit previously set in the storage within the measuring device, and
setting the second data transmission schedule time at a smaller value according as the difference is larger.

21. The living body information measuring system according to Claim 19, wherein
the measuring device has a function of setting the second data transmission schedule time at a larger value according as the dispersion of the measurement data is smaller, and
setting the second data transmission schedule time at a smaller value according as the dispersion is larger.

22. The living body information measuring system according to Claim 19, wherein
the measuring device has a function of setting the second data transmission schedule time at a larger value according as the variation rate of the measurement data is smaller, and
setting the second data transmission schedule time at a smaller value according as the variation rate is larger.

23. The living body information system according to Claim 19, wherein the measuring device has a function of determining the second data transmission schedule time based on a time of measuring the measurement data.

24. The living body information measuring system according to Claim 19, wherein the data collecting device transmits the second data transmission schedule time received from the measuring device together with the data transmission schedule time, to the measuring device.

25. The living body information measuring system according to Claim 3, wherein the living body information measuring sensor of the measuring device detects a pulse.

26. The living body information measuring system according to Claim 3, wherein the living body information measuring sensor of the measuring device detects acceleration of a living body's movement.

27. The living body information measuring system according to Claim 3, wherein the living body information measuring sensor of the measuring device detects a breathing rate.

28. The living body information measuring system according to Claim 3, wherein the data collecting device is connected by one and more external lines.

29. A command transmission method of the data collecting device, **characterized by** comprising
a step of calculating the first data transmission schedule time to the measuring device,
a step of including a value of the first data transmission schedule time in the data transfer request command, and
a step of transmitting a data transfer request command.

30. A controlling method of the measuring device, **characterized by** comprising
a step of receiving the data transfer request command transmitted from the data collecting device,
a step of transmitting the requested measurement data, and
a step of cutting off power of the receiver, until the first data transmission schedule time of the data transfer request command, after transmission of the living body information data.

31. A data collecting device for use in the system of claim 1, **characterized by** comprising
calculating means for calculating the first data transmission schedule time to the measuring device, and
command creating means for including the value of the first data transmission schedule time in the data transfer request command.

32. A measuring device for use in the system of claim 1, **characterized by**
a receiver for receiving the data transfer request command transmitted from the data collecting device,
a transmitter for transmitting the requested measurement data, and
a power on/off circuit for cutting off the power of the receiver until the first data transmission schedule time of the data transfer request command.

33. A measuring device for use in the system of claim 1, **characterized by** comprising
transmission schedule time calculating means for calculating the second data transmission schedule time,
living body information data creating means for including the value of the second data transmission schedule time in the living body information data, and
controlling means for cutting off power of a transmitter/receiver, until the first data transmission schedule time, after transmission of the living body information data.
